# EUROPÄISCHE PATENTSCHRIFT

(11) **EP 1 022 282 B1**
(45) Veröffentlichungstag und Bekanntmachung des Hinweises auf die Patenterteilung: **20.10.2004**
(21) Anmeldenummer: 00100475.3
(22) Anmeldetag: 11.01.2000
(51) Int. Cl.: C07F 15/00, C07C 6/04

(54) **Homo- und heterobimetallische Alkylidenkomplexe des Rutheniums mit N-heterocyclischen Carbenliganden und deren Anwendung als hochaktive, selektive Katalysatoren für die Olefin-Metathese**
Homo- and hetero metal alkylidene complexes of ruthenium with N-heterocyclic carbene ligands, and their use as highly active and selective catalysts for olefin metathesis
Complexes homo- ou hétérométalliques de ruthénium et des ligandes carbéniques N-hétérocycliques, et leur utilisation comme catalyseurs actifs et selectifs pour la métathèse d'oléfines

(30) Priorität: 22.01.1999 DE 19902439
(43) Veröffentlichungstag der Anmeldung: 26.07.2000
(73) Patentinhaber: Degussa AG, 40474 Düsseldorf (DE)
(72) Erfinder: Herrmann, Wolfgang Anton, Prof. Dr., 85354 Freising (DE); Kohl, Florian J., 80798 München (DE); Weskamp, Thomas, Symyx Technologies, Santa Clara, CA 95051 (US)

(56) Entgegenhaltungen:
- WESKAMP, T. ET AL.: "a novel class of ruthenium catalysts for olefin metathesis" ANGEWANDTE CHEMIE INTERNATIONAL EDITION, Bd. 37, Nr. 18, 1998, Seiten 2490-2493, XP002168987

## Beschreibung

Die Erfindung betrifft homo- und heterobimetallische Alkylidenkomplexe des Rutheniums mit N-heterocyclischen Carbenliganden und ein Verfahren zur Herstellung von Olefinen durch Olefin-Metathese aus acyclischen Olefinen mit zwei oder mehr Kohlenstoffatomen oder/und aus cyclischen Olefinen mit drei oder mehr Kohlenstoffatomen, wobei mindestens eine dieser bimetallischen Alkylidenkomplexverbindungen als Katalysator eingesetzt wird.

Übergangsmetallkatalysierte C-C-Verknüpfungen gehören zu den wichtigsten Reaktionen der organischen Synthesechemie. In diesem Zusammenhang stellt die Olefin-Metathese einen wesentlichen Bestandteil dar, da mittels dieser Reaktion nebenproduktfrei Olefine synthetisiert werden können. Die Olefin-Metathese besitzt dabei nicht nur hohes Potential auf dem Sektor der präparativen, organischen Synthese wie z.B. für die Ringschlußmetathese (RCM), die Ethenolyse oder die Metathese acyclischer Olefine, sondern auch in der Polymerchemie wie z.B. für die ringöffnende Metathesepolymerisation (ROMP), die acyclische Dienmetathese (ADMET) oder Alkinpolymerisation.

Seit ihrer Entdeckung in den 50er Jahren konnten mehrere großtechnische Prozesse realisiert werden. Dennoch avancierte die Olefin-Metathese erst in jüngster Zeit durch die Entdeckung neuer Katalysatoren zu einer breit anwendbaren Synthesemethode (Übersichtsartikel siehe: J. C. Mol in: B. Cornils, W. A. Herrmann, Applied Homogeneous Catalysis with Organometallic Compounds, VCH, Weinheim, 1996, S.318-332; M. Schuster, S. Blechert, Angew. Chem. 1997, 109, 2124-2144).

Zahlreiche, grundlegende Arbeiten haben wesentlich zum Verständnis dieser übergangsmetallkatalysierten Reaktion beigetragen, bei der ein Austausch von Alkylideneinheiten zwischen Olefinen erfolgt. Der allgemein akzeptierte Mechanismus beinhaltet Metallalkylidenkomplexe als aktive Spezien. Diese reagieren mit Olefinen zu Metallacyclobutanintermediaten, die unter Cycloreversion wieder Olefine und Alkylidenkomplexe generieren. Die Isolierung von metatheseaktiven Alkyliden- und Metallacyclobutankomplexen untermauern diese mechanistischen Vorstellungen.

Zahlreiche Beispiele finden sich vor allem in der Komplexchemie des Molybdäns und Wolframs. Speziell durch Arbeiten von Schrock wurden wohldefinierte Alkylidenkomplexe erhalten, die in ihrer Reaktivität kontrollierbar sind (J. S. Murdzek, R. R. Schrock, Organometallics 1987, 6, 1373-1374). Die Einführung einer chiralen Ligandsphäre an diesen Komplexen ermöglichte die Synthese von Polymeren mit hoher Taktizität (K. M. Totland, T. J. Boyd, G. C. Lavoie, W. M. Davis, R. R. Schrock, Macromolecules 1996, 29, 6114-6125). Chirale Komplexe gleichen Strukturtyps wurden auch in der Ringschluß-Metathese mit Erfolg eingesetzt (O. Fujimura, F. J. d. I. Mata, R. H. Grubbs, Organometallics 1996, 15, 1865-1871; J. B. Alexander, D. S. La, D. R. Cefalo, A. H. Hoveyda, R. R. Schrock, J. Am. Chem. Soc. 1998, 120, 4041-4042). Nachteilig stellt sich jedoch die hohe Empfindlichkeit gegenüber funktionellen Gruppen, Luft und Wasser heraus.

In jüngster Zeit haben sich phosphanhaltige Komplexsysteme des Rutheniums etabliert (R. H. Grubbs, S. T. Nguyen, L. K. Johnson, M. A. Hillmyer, G. C. Fu, WO 96/04289, 1994; P. Schwab, M. B. France, J. W. Ziller, R. H. Grubbs, Angew. Chem., 1995, 107, 2179-2181; Angew. Chem. Int. Ed. Engl. 1995, 34, 2039-2041; R. H. Grubbs, E. L. Dias, Organometallics, 1998, 17, 2758). Aufgrund des elektronenreichen, "weichen" Charakters später Übergangsmetalle besitzen diese Komplexe eine hohe Toleranz gegenüber harten, funktionellen Gruppen. Dies wird beispielsweise durch ihren Einsatz in der Naturstoffchemie (RCM von Dienen) demonstriert (Z. Yang, Y. He, D. Vourloumis, H. Vallberg, K. C. Nicolaou, Angew. Chem. 1997, 109, 170-172; Angew. Chem., Int. Ed. Engl. 1997, 36, 166-168; D. Meng, P. Bertinato, A. Balog, D. S. Su, T. Kamenecka, E. J. Sorensen, S. J. Danishefsky, J. Am. Chem. Soc. 1997, 119, 2733-2734; D. Schinzer, A. Limberg, A. Bauer, O. M. Böhm, M. Cordes, Angew. Chem. 1997, 109, 543-544; Angew. Chem., Int. Ed. Engl. 1997, 36, 523-524; A. Fürstner, K. Langemann, J. Am. Chem. Soc. 1997, 119, 9130-9136).

Die Variationsbreite der verwendeten Phosphanliganden ist jedoch aufgrund sterischer und elektronischer Faktoren sehr limitiert. Lediglich stark basische, sterisch anspruchsvolle Alkylphosphane wie Tricyclohexyl-, Triisopropyl- und Tricyclopentylphosphan eignen sich für die Metathese acyclischer Olefine und wenig gespannter Ringsysteme. Demnach sind diese Katalysatoren nicht in ihrer Reaktivität einstellbar. Auch chirale Komplexe dieses Strukturtyps konnten nicht realisiert werden.

Es konnte von den Erfindern der vorliegenden Erfindung bereits gezeigt werden, daß durch die Einführung von N-heterocyclischen Carbenen als Liganden nicht nur die Aktivität dieser Systeme gesteigert werden kann, sondern auf Grund der wesentlich variableren Ligandsphäre auch neuartige Steuermöglichkeiten wie z.B. in bezug auf Chiralität, Taktizität oder Regulierung der Aktivität zugänglich werden (T. Weskamp, W. C. Schattenmann, M. Spiegler, W. A. Herrmann, Angew. Chem. 1998, 110, 2631-2633; Angew. Chem. Int. Ed. Engl. 1998, 37, 2490-2493).

Die Toleranz gegenüber funktionellen Gruppen wird aber in allen Rutheniumsystemen nach wie vor mit einer im Vergleich zu Molybdän und Wolfram deutlich verminderten Aktivität bezahlt.

Aus diesen Gründen bestand die Aufgabe, maßgeschneiderte Metathesekatalysatoren zu entwickeln, die sich neben ihrer hohen Toleranz gegenüber funktionellen Gruppen und einer variableren Ligandsphäre durch deutlich gesteigerte Aktivitäten auszeichnen.

Die Aufgabe wird erfindungsgemäß gelöst durch eine Komplexverbindung des Rutheniums der allgemeinen Strukturformel I, 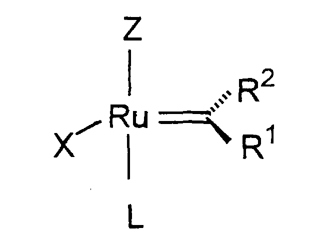 in der X ein anionischer Ligand ist,
in der Z einen ein- bis dreizähnigen Liganden darstellt, der ein Metall enthält und nicht-ionisch an das Rutheniumzentrum gebunden ist,
in der R¹ und R² gleich oder unabhängig voneinander verschieden sind, aber auch einen Cyclus ausbilden können,
in der R¹ und R² für Wasserstoff oder/und für eine Kohlenwasserstoffgruppe stehen, wobei die Kohlenwasserstoffgruppen gleich oder unabhängig voneinander verschieden aus einem geradkettigen oder verzweigten oder/und cyclischen Rest aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen, Arylresten mit 6 bis 30 Kohlenstoffatomen und Silylresten bestehen,
wobei in den Kohlenwasserstoff- oder/und Silylgruppen die Wasserstoffatome teilweise oder gänzlich durch eine Alkyl-, Aryl-, Alkenyl-, Alkinyl-, Metallocenyl-, Halogen-, Nitro-, Nitroso-, Hydroxy-, Alkoxy-, Aryloxy-, Amino-, Amido-, Carboxyl-, Carbonyl-, Thio- oder/und Sulfonylgruppe einfach oder mehrfach, gleich oder unabhängig voneinander verschieden ersetzt sein können,
in der Ligand L ein N-heterocyclisches Carben der allgemeinen Formeln II - V ist, 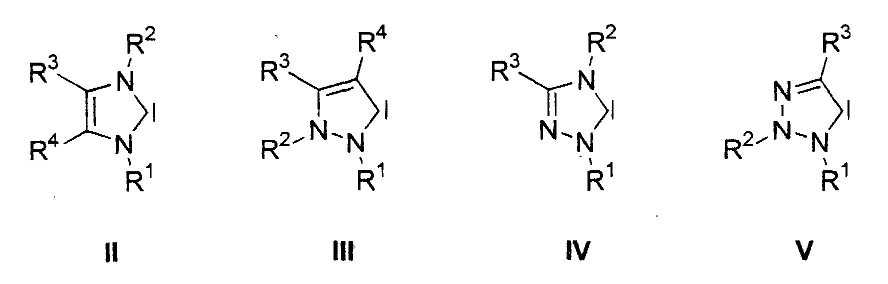 wobei R¹, R², R³ und R⁴ in den Formeln II, III, IV und V gleich oder unabhängig voneinander verschieden für Wasserstoff oder/und für eine Kohlenwasserstoffgruppe stehen,
wobei die Kohlenwasserstoffgruppen gleich oder unabhängig verschieden voneinander aus cyclischen, geradkettigen oder/und verzweigten Resten aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen, Arylresten mit 6 bis 30 Kohlenstoffatomen bestehen, bei denen gegebenenfalls mindestens ein Wasserstoff durch funktionelle Gruppen ersetzt sein kann, und wobei gegebenenfalls R³ und R⁴ für Halogen-, Nitro-, Nitroso-, Alkoxy-, Aryloxy-, Amido-, Carboxyl-, Carbonyl-, Thio-, Silyl- oder/und Sulfonylgruppe einfach oder mehrfach, gleich oder unabhängig voneinander verschieden stehen kann.

Die erfindungsgemäß aufgebauten Alkylidenkomplexverbindungen mit einem N-heterocyclischen Carbenliganden in Kombination mit einem ein- bis dreizähnigen Liganden Z sind hochaktive Katalysatoren für die Olefin-Metathese. Sie sind besonders kostengünstig. Die Olefin-Metathese mit den erfindungsgemäßen Katalysatoren zeichnet sich neben einer hohen Toleranz gegenüber unterschiedlichsten funktionellen Gruppen und ihrer Variationsvielfalt in der Ligandensphäre insbesondere durch eine große Aktivität aus.

Durch Variation des präparativ einfach zugänglichen N-heterocyclischen Carbenliganden L können Aktivität und Selektivität gezielt gesteuert, und darüber hinaus kann Chiralität auf einfache Art und Weise eingeführt werden.

So können in den allgemeinen Formeln II, III, IV und V der Wasserstoff in den Kohlenwasserstoffgruppen R¹, R², R³ und R⁴ teilweise oder gänzlich durch Halogen-, insbesondere Chlor, Brom oder Jod, Nitro-, Nitroso-, Hydroxy-, Aryloxy-, Amino-, Amido-, Carboxyl-, Carbonyl-, Thio-, Sulfonyl-, oder/und Metallocenylgruppen einfach oder mehrfach, gleich oder unabhängig voneinander verschieden ersetzt sein.

Beispiele für funktionelle Gruppen als geeignete substituierte Gruppen R¹, R², R³ und R⁴ können sein Hydroxymethyl, 2-Hydroxyethyl, 3-Hydroxypropyl, Aminomethyl, 2-Aminoethyl, 3-Aminopropyl, 2-Acetylethylaminoethyl, Ethoxyethyl, Polyether, Ethoxyacetyl, Methoxycarbonylethyl, Ethoxycarbonylethyl, Kaliumcarboxylatomethyl und Isopropylaminocarbonylmethyl.

R¹ und R² können beispielsweise ausgewählt werden unter Resten Methyl, Ethyl, Isopropyl, Cyclopropyl, Cyclohexyl, 1-Phenylethyl, 1-Naphthylethyl, 1-tert.-Butylethyl, tert. Butyl, Phenyl, gegebenfalls substituiert mit Nitro-, Amino-, Hydroxy- oder/und Carboxylgruppen, Mesityl, Tolyl und Naphthyl. Soweit Chiralität gegeben ist, können die Reste auch in (R) und (S)-Form vorliegen.

Als Beispiele für R³ und R⁴ können Wasserstoff, Methyl, Ethyl und Phenyl, gegebenenfalls substituiert mit einer Nitro-, Amino- Hydroxy oder/und Carboxylgruppe genannt werden. In diesen Formeln können R³ und R⁴ auch ein anneliertes Ringsystem bilden.

Die Liganden L der allgemeinen Formeln II, III, IV oder/und V können zentrale, axiale oder/und planare Chiralität aufweisen.

In der allgemeinen Formel steht Z für einen ein- bis dreizähnige Liganden L'ₙMX'ₘ wobei n = 0 bis 4, m = 0 bis 6 und m + n ≠ 0 ist, L'gleich oder unabhängig verschieden voneinander ausgewählt sein kann unter π-gebundenen, ungesättigten carbocyclischen Kohlenwasserstoffen und neutralen Elektronendonoren, X' gleich oder unabhängig verschieden voneinander für einen anionischen Liganden aus der Gruppe von Halogenid, Pseudohalogenid, Tetraphenylborat, perhalogeniertes Tetraphenylborat, Tetrahalogenoborat, Hexahalogenophosphat, Hexahalogenoantimonat, Trihalogenmethansulfonat, Alkoxid, Thiolat, Carboxylat, Tetrahalogenoaluminat, Tetracarbonylcobaltat, Hexahalogenoferrat(III), Tetrahalogenoferrat(III) oder/und Tetrahalogenopalladat(II) stehen kann und M für ein Metall steht.

Bevorzugte Beispiele für L' sind Cyclopentadienyl-, Pentamethylcyclopentadienyl- oder anders substituerte Cyclopentadienylreste, Benzol und substituierte Benzole, beispielsweise Cymol, sowie Phosphane, Phosphite, Amine, Imine, Nitrile, N-heterocyclische Carbene und Carbonyl.

Bevorzugte Beispiele für X' sind Halogenid, insbesondere Chlor, Brom oder Jod, Pseudohalogenid, Alkoxid, Thiolat, Amid- und Carboxylat.
Stellt das Halgonid einen Substituenten in einer der vorstehend genannten Verbindungen dar, ist Chlorid bevorzugt.

Das Metall M kann ausgewählt sein unter einem Metall der 1. bis 8. Nebengruppe und einem Metall der 1. bis 4. Hauptgruppe, insbesondere einem Metall der 1., 2., 6., 7. und 8. Nebengruppe sowie der 1. bis 4. Hauptgruppe, wobei Metalle der 8. Nebengruppe besonders bevorzugt sind. Bevorzugte Beispiele sind Os, Ru, Ir, Rh, Fe und Pd für die 8. Nebengruppe, Re für die 7. Nebengruppe, Mo und W für die 6. Nebengruppe und B, Al und Si für die 3. und 4. Hauptgruppe.

Der anionische Ligand X der erfindungsgemäßen Komplexverbindung ist vorzugsweise ein Halogenid, Pseudohalogenid, Tetraphenylborat, perhalogeniertes Tetraphenylborat, Tetrahalogenoborat, Hexahalogenophosphat, Hexahalogenoantimonat, Trihalogenomethansulfonat, Alkoxid, Phenolat, Thiolat, Carboxylat, Tetrahalogenoaluminat, Tertracarbonyl-Cobaltat, Hexahalogenoferrat(III), Tetrahalogenoferrat(III) oder/und Tetrahalogenopalladat(II), wobei Halogenid, Pseudohalogenid, Tetraphenylborat, perfluoriertes Tetraphenylborat, Tetrafluoroborat, Hexafluorophosphat, Hexafluoroantimonat, Trifluormethansulfonat, Alkoxid, Phenolat, Carboxylat, Tetrachloroaluminat, Tertracarbonyl-Cobaltat, Hexafluoroferrat(III) Tetrachloroferrat(III) oder/und Tetrachloropalladat(II) bevorzugt sind und wobei unter den Pseudohalogeniden Cyanid, Rhodanid, Cyanat, Isocyanat, Thiocyanat und Isothiocyanat bevorzugt sind, wobei als Halogenid vorzugsweise Chlor, Brom oder auch Jod eingesetzt werden.

Vorzugsweise weisen die Alkylreste, Alkenylreste, Alkinylreste bzw. die Alkylenreste, Alkenylenreste, Alkinylenreste in den Formeln I bis VI 1 bzw. 2 bis 20 Kohlenstoffatome, besonders bevorzugt 1 bzw. 2 bis 12 Kohlenstoffatome, auf.

In der allgemeinen Strukturformel I der Komplexverbindung stehen R¹ bis R² bevorzugt für Wasserstoff, substituierte oder/und nichtsubstituierte Alkyl-, Alkenyl- oder/und Arylreste, X bevorzugt für Halogenid-, Alkoxid- oder/und Carboxylationen sind und L bevorzugt für ein N-heterocyclisches Carben der allgemeinen Formel II.

Die Synthese der Komplexe erfolgt überlicherweise durch Ligandensubstitution entsprechender Phosphankomplexe. Diese können entsprechend der Reaktionsgleichung in einem ersten Schritt selektiv einfach aber auch zweifach substituiert werden und anschließend in einem zweiten Schritt beispielsweise mit dem entsprechendem Dimeren von Z zur erfindungsgemäßen Komplexverbindung reagieren:

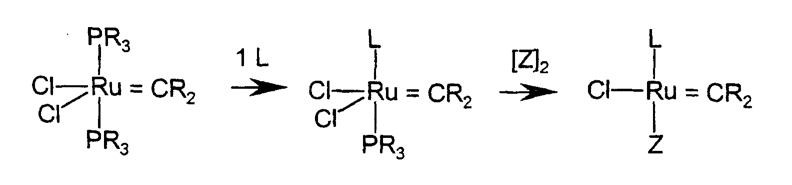

Die erfindungsgemäßen Komplexverbindungen erweisen sich als äußerst effiziente Katalysatoren in der Olefin-Metathese. Die ausgezeichnete Metatheseaktivität wird durch mehrere Beispiele verschiedener metathetischer Reaktionen in den Beispielen demonstriert.

Deshalb umfaßt diese Erfindung auch die Verfahren aller Olefin-Metathese-Reaktionen wie Ringöffnende Metathesepolymerisation (ROMP), Metathese acyclischer Olefine, Ethanolyse, Ringschlußmetathese (RCM), acyclische Dien-Metathese-Polymerisation (ADMET) und Depolymerisation olefinischer Polymere. Die hohe Stabilität und Toleranz der erfindungsgemäßen Komplexverbindungen gegenüber funktionellen Gruppen, insbesondere Gruppen von Alkoholen, Aminen, Thiolen, Ketonen, Aldehyden, Carbonsäuren, Estern, Amiden, Ethern, Silanen, Sulfiden und Halogenen erlaubt die Anwesenheit derartiger funktioneller Gruppen während der Metathesereaktion.

Die Aufgabe wird ferner durch ein Verfahren zur Herstellung von acyclischen Olefinen mit zwei oder mehr Kohlenstoffatomen oder/und von cyclischen Olefinen mit drei oder mehr Kohlenstoffatomen jeweils entsprechend der allgemeinen Formel VI 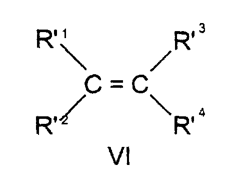 aus acyclischen Olefinen mit zwei oder mehr Kohlenstoffatomen oder/und aus cyclischen Olefinen mit drei oder mehr Kohlenstoffatomen jeweils entsprechend der allgemeinen Formel VI durch Olefin-Metathese-Reaktion in Gegenwart mindestens einer der vorstehend beschriebenen Komplexverbindungen gelöst, wobei R'¹,R'², R'³ und R'⁴ in der allgemeinen Formel VI für Wasserstoff oder/und Kohlenwasserstoffgruppen stehen,
wobei die Kohlenwasserstoffgruppe aus gleich oder unabhängig voneinander verschieden geradkettigen, verzweigten oder/und cyclischen Resten aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen, Arylresten mit 6 bis 30 Kohlenstoffatomen, Metallocenyl- oder/und Silylresten besteht, bei denen gegebenenfalls mindestens ein Wasserstoff durch eine funktionelle Gruppe ersetzt sein kann,
wobei gegebenenfalls R'¹, R'², R'³ und R'⁴ für Halogen-, Nitro-, Nitroso-, Hxdroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden stehen können.

Vorzugsweise sind in den eingesetzten oder/und in den herzustellenden Olefinen eine oder/und mehrere Doppelbindungen enthalten. Insbesondere können R'¹, R'², R'³ und R'⁴ in den Olefinen der allgemeinen Formel VI paarweise einen Cyclus ausbilden.

In den Olefinen der allgemeinen Formel VI kann der Wasserstoff in den Kohlenwasserstoffgruppen R'¹,R'², R'³ und R'⁴ teilweise oder gänzlich durch Halogen-, Silyl-, Nitro-, Nitroso-, Hxdroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden ersetzt.

Für R'¹, R'², R'³ und R'⁴ sowie für Substituenten des Wasserstoffs können die gleichen Beispiele genannt werden wie in bezug auf die Formeln I bis V vorstehend aufgeführt.

Bei dem erfindungsgemäßen Verfahren kann der Prozeß mit oder ohne Lösungsmittel, jedoch vorzugsweise mit organischen Lösungsmitteln, durchgeführt werden. Das erfindungsgemäße Verfahren kann unter Zusatz einer Brönstedt-Säure, vorzugsweise von HCl, HBr, HI, HBF₄, HPF₆, oder/und Trifluoressigsäure, oder/und unter Zusatz einer Lewis-Säure, vorzugsweise von BF₃, AlCl₃ oder/und Znl₂, durchgeführt werden.
Damit wird es erstmals überraschenderweise möglich, mit großer Katalysatoraktivität die verschiedensten Olefine individuell auf unterschiedliche Eigenschaften aufgrund einfacher Variation der Katalysebedingungen oder/und der Katalysatoren maßgeschneidert zu erhalten, nicht zuletzt da das erfindungsgemäße Verfahren zur Herstellung von Olefinen eine unerwartet hohe Toleranz gegenüber funktionellen Gruppen aufweist.

### Beispiele

Die folgenden Beispiele belegen die Erfindung, schränken sie aber nicht in ihrer Breite ein.

### 1) Herstellung der erfindungsgemäßen Komplexverbindung

### Allgemeine Arbeitsvorschrift:

1 mmol RuCl₂(di-R-imidazolin-2-yliden)₂(CHPh) oder RuCl₂(di-R-imidazolin-2-yliden)(PCy₃)(CHPh) worin R für einen beliebigen Rest steht, werden in 5 ml CH₂Cl₂ gelöst und mit einer Lösung von 1 mmol [L'MX'₂]₂ in 5 ml CH₂Cl₂ versetzt. Die Reaktionslösung wird etwa 15 bis 180 min bei Raumtemperatur RT gerührt, anschließend wird das Lösungsmittel entfernt, der Komplex mit einem Toluol/Pentan-Gemisch gewaschen und mehrere Stunden im Hochvakuum getrocknet.
Die Umsetzungen verlaufen in den angegebenen Zeiträumen quantitativ.

Nach der angegebenen allgemeinen Arbeitsvorschrift wurden folgende Verbindungen hergestellt:
Katalysator 1) 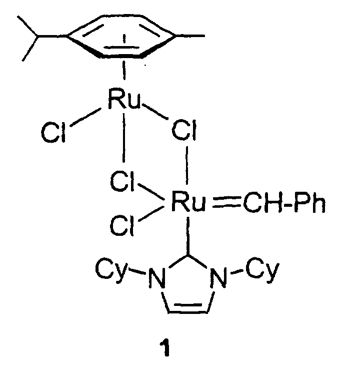 Edukte: RuCl₂(di-cyclohexyl-imidazolin-2-yliden)(PCy₃)(CHPh) und [(p-Cymol)RuCl₂]₂
Reaktionszeit: 2 h

| | | | | |
|---|---|---|---|---|
| Elementaranalyse für C₃₂H₄₄Cl₄N₂Ru₂ | berechnet | C 48.00, | H 5.54, | N 3.50 |
| | gefunden | C 48.11; | H 5.61; | N 3.52. |

¹H NMR (CD₂Cl₂ / 25 °C): δ = 21.14 (1H, s, Ru=CH), 7.89 (2H, d, ³*J*_{HH} = 7.8 Hz, o-H von C₆H₅), 7.67 (1H, t, ³*J*_{HH} = 7.8 Hz, *p*-H von C₆H₅), 7.22 (2H, t, ³*J*_{HH} = 7.8 Hz, *m*-H von C₆H₅), 7.09 (1H, s, NCH), 6.65 (1H, s, NCH), 5.70 (1H, m, CH von NC₆H₁₁), 5.53, 5.50, 5.43, und 5.28 (alle 1H, d, ³*J*_{HH} = 5.7 Hz, CH von p-Cymol) 3.05 (1H, m, CH von NC₆H₁₁), 2.85 (1H, m, C*H*(CH₃)₂ von p-Cymol), 2.34 (3H, s, CH₃ von p-Cymol), 1.82-0.91 (20H, alle m, CH₂ von NC₆H₁₁), 1.41 (3H, d, ³*J*_{HH} = 7.0 Hz, CH(C*H*₃)₂ von p-Cymol), 1.27 (3H, d, ³*J*_{HH} = 7.0 Hz, CH(C*H*₃)₂ von p-Cymol). ¹³C NMR (CD₂Cl₂/25 °C): δ = 319.4 (Ru=CH), 165.2 (NCN), 154.0 (*ipso*-C von C₆H₅), 131.4, 130.7, und 128.7 (*o*-C, *m*-C, und *p*-C von C₆H₅), 119.1 und 118.0 (NCH), 101.3, 96.8, 81.3, 80.6, 79.7, und 79.4 (p-Cymol), 58.9 und 56.7 (CH von NC₆H₁₁), 36.0, 34.9, 31.3, 25.8, 25.4, und 22.3 (CH₂ von NC₆H₁₁), 30.8 (*C*H(CH₃)₂ von p-Cymol), 22.2 und 21.9 (CH(*C*H₃)₂ von p-Cymol), 18.8 (CH₃ von p-Cymol).
Katalysator 2) 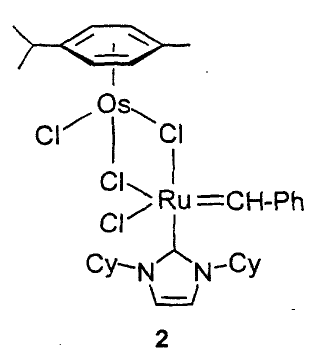 Edukte: RuCl₂(di-cyclohexyl-imidazolin-2-yliden)₂(CHPh) und [(p-Cymol)OsCl₂]₂
Reaktionszeit: 3 h

| | | | | |
|---|---|---|---|---|
| Elementaranalyse für C₃₂H₄₄Cl₄N₂OsRu | berechnet | C 43.14, | H 4.98, | N 3.15 |
| | gefunden | C 43.31; | H 5.11; | N 3.13. |

¹H NMR (CD₂Cl₂ / 25 °C): δ = 21.21 (1H, s, Ru=CH), 7.91 (2H, d, ³*J*_{HH} = 6.4 Hz, *o*-H von C₆H₅), 7.72 (1H, t, ³*J*_{HH}= 6.4 Hz, *p*-H von C₆H₅), 7.24 (2H, t, ³*J*_{HH}= 6.4 Hz, *m*-H von C₆H₅), 7.04 (1H, s, NCH), 6.69 (1H, s, NCH), 5.70 (1H, m, CH von NC₆H₁₁), 6.08( 1H, d, ³*J*_{HH} = 5.9 Hz, CH von p-Cymol), 5.95 (1H, d, ³*J*_{HH}= 5.9 Hz, CH von p-Cymol) 5.75 (2H, app t, ³*J*_{HH} = 5.9 Hz, CH von p-Cymol), 3.07 (1H, m, CH von NC₆H₁₁), 2.83 (1H, m, C*H*(CH₃)₂ von p-Cymol), 2.34 (3H, s, CH₃ von p-Cymol), 1.90-0.85 (20H, alle m, CH₂ von NC₆H₁₁), 1.39 (3H, d, ³*J*_{HH} = 6.8 Hz, CH(C*H*₃)₂ von p-Cymol), 1.33 (3H, d, ³*J*_{HH} = 6.8 Hz, CH(C*H*₃)₂ von p-Cymol). ¹³C NMR (CD₂Cl₂ /25 °C): δ = 319.7 (Ru=CH), 165.0 (NCN), 153.9 (*ipso*-C von C₆H₅), 131.2, 130.7, und 128.6 (*o*-C, *m*-C, und *p*-C von C₆H₅), 119.3 und 118.1 (NCH), 96.5, 91.5, 71.6, 71.4, 70.4, und 69.7 (p-Cymol), 58.8 und 56.5 (CH von NC₆H₁₁), 35.8, 35.3, 31.2, 25.9, 25.2, und 22.7 (CH₂ von NC₆H₁₁), 31.2 (CH(CH₃)₂ von p-Cymol), 22.2 und 22.1 (CH(*C*H₃)₂ von p-Cymol), 18.7 (CH₃ von p-Cymol).
Katalysator 3) 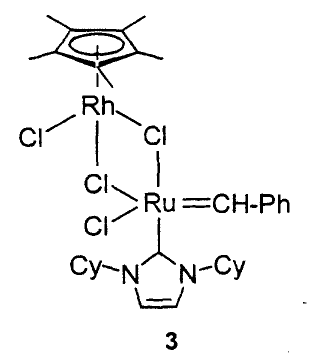 Edukte: RuCl₂(di-cyclohexyl-imidazolin-2-yliden)₂(CHPh) und [(Cp*RhCl₂]₂
Reaktionszeit: 15 min

| | | | | |
|---|---|---|---|---|
| Elementaranalyse für C₄₅H₄₆Cl₂N₄RhRu | berechnet | C 47.88, | H 5.65, | N 3.49 |
| | gefunden | C 47.99, | H 5.70, | N 3.45. |

¹H-NMR (CD₂Cl₂/25 °C): δ = 21.20 (1H, s, Ru=CH), 7.95 (2H, d, ³*J*_{HH} = 7.2 Hz, *o*-H von C₆H₅), 7.67 (1H, t, ³*J*_{HH} = 7.2 Hz, *p*-H von C₆H₅), 7.25 (2H, t, ³*J*_{HH} = 7.8 Hz, *m*-H von C₆H₅), 7.09 (1H, s, NCH), 6.68 (1H, s, NCH), 6.57 (1H, m, CH von NC₆H₁₁), 2.97 (1H, m, CH von NC₆H₁₁), 1.85-0.86 (20H, alle m, CH₂ von NC₆H₁₁), 1.74 (15H, s, CH₃ von Cp*). ¹³C NMR (CD₂Cl₂ /25 °C): δ = 319.3 (Ru=CH), 164.4 (NCN), 153.5 (*ipso*-C von C₆H₅), 131.2, 130.4, und 128.7 (*o*-C, *m*-C, und *p*-C von C₆H₅), 118.9 und 118.3 (NCH), 94.3 (d, *J*_{RhC} = 7.5 Hz, CCH₃ von Cp*), 58.3 und 56.4 (CH von NC₆H₁₁), 35.2, 34.1, 33.3, 25.8, 22.4, 21.2 (CH₂ von NC₆H₁₁), 9.31(CH₃ von Cp*),

### 2 - 4) Anwendung der erfindungsgemäßen Komplexverbindung bei der Olefin-Metathese

Die im folgenden aufgeführten Beispiele demonstrieren das Potential der erfindungsgemäßen Komplexverbindungen in der Olefinmetathese. Der Vorteil dieser erfindungsgemäßen Komplexverbindungen verglichen mit bekannten phosphanhaltigen Systemen liegt in der deutlich gesteigerten Aktivität insbesondere in der Ringöffnenden Metathesepolymerisation. Dadurch können Olefine effektiv metathetisiert werden, die sonst nicht oder nur schwer einer Metathesereaktion unterzogen werden können.

### 2) Ringöffnende Metathesepolymerisation

Als Beispiele dienen Norbornen, funktionalisiertes Norbornen, 1,5-Cyclooctadien und Cyclopenten

### 2a) Ringöffnende Metathesepolymerisation von Norbornen

Zur Demonstration der Aktivität wurde Norbornen einer Ringöffnenden Metathesepolymerisation unterzogen.

### Typischer Reaktionsansatz:

In einem Kolben werden 1.0 µmol der jeweiligen Komplexverbindung in 30 ml CH₂Cl₂ gelöst. Die Reaktion wird durch Zugabe von 20.0 mmol Norbornen gestartet und nach gewisser Zeit durch Eingießen der Reaktionslösung in 500 ml Methanol (Ausfällen des gebildeten Polynorbornenamers) gestoppt. Das ausgefällte Polynorbornenamer wird durch Filtration isoliert und nach mehrmaligem Umfällen aus CH₂Cl₂/ Methanol oder Toluol / Methanol im Hochvakuum bis zur Gewichtskonstanz getrocknet. Die Ausbeutebestimmung erfolgt gravimetrisch.

Die Katalysatoren, Reaktionszeiten, Ausbeuten und Turnover Frequencies (TOF) sind in Tab. 1 angegeben.

**Tab. 1**

| ROMP von Norbornen mit erfindungsgemäßen Komplexverbindungen. 2b) Ringöffnende Metathesepolymerisation von funktionalisierten Norbornenderivaten | | | | |
|---|---|---|---|---|
| Katalysator | Monomer / Katalysator | Zeit [s] | Ausbeute [%] | TOF [h⁻¹] |
| 1 | 20000 | 60 | 76 | 9 · 10⁵ |
| 2 | 20000 | 60 | 82 | 1 · 10⁶ |
| 3 | 20000 | 20 | 80 | 3 · 10⁶ |

Zur Demonstration der Aktivität und der Toleranz gegenüber funktionellen Gruppen wurde Essigsäure-5-norbomen-2-yl-ester einer Ringöffnenden Metathesepolymerisation unterzogen.

### Typischer Reaktionsansatz:

In einem Kolben werden 1.0 µmol der jeweiligen Komplexverbindung in 2 ml CH₂Cl₂ gelöst. Die Reaktion wird durch Zugabe von 5.0 mmol Essigsäure-5-norbomen-2-ylester gestartet und nach gewisser Zeit durch Eingießen der Reaktionslösung in 500 ml Methanol (Ausfällen des gebildeten Polynorbomenamers) gestoppt. Das ausgefällte Polynorbornenamer wird durch Filtration isoliert und nach mehrmaligem Umfällen aus CH₂Cl₂/ Methanol oder Toluol / Methanol im Hochvakuum bis zur Gewichtskonstanz getrocknet. Die Ausbeutebestimmung erfolgt gravimetrisch.

Die Katalysatoren, Reaktionszeiten, Ausbeuten und TOFs sind in Tab. 2 angegeben.

**Tab. 2**

| ROMP von Essigsäure-5-norbornen-2-yl-ester mit erfindungsgemäßen Komplexverbindungen. | | | | |
|---|---|---|---|---|
| Katalysator | Monomer / Katalysator | Zeit [s] | Ausbeute [%] | TOF [h⁻¹] |
| 1 | 5000 | 180 | 95 | 1 · 10⁵ |
| 2 | 5000 | 180 | 95 | 1 · 10⁵ |
| 3 | 5000 | 60 | 80 | 2 · 10⁵ |

### 2c) Ringöffnende Metathesepolymerisation von 1,5-Cyclooctadien

Zur Demonstration der Aktivität der erfindungsgemäßen Komplexverbindungen wurden NMR-spektroskopisch kontrollierte Kinetiken der Ringöffnenden Metathesepolymerisation von 1,5-Cyclooctadien aufgenommen. Aufgrund seiner gegenüber Norbornen deutlich verringerten Ringspannung gilt 1,5-Cyclooctadien bereits als äußerst schwierig zu polymerisierendes Substrat.

### Typischer Reaktionsansatz:

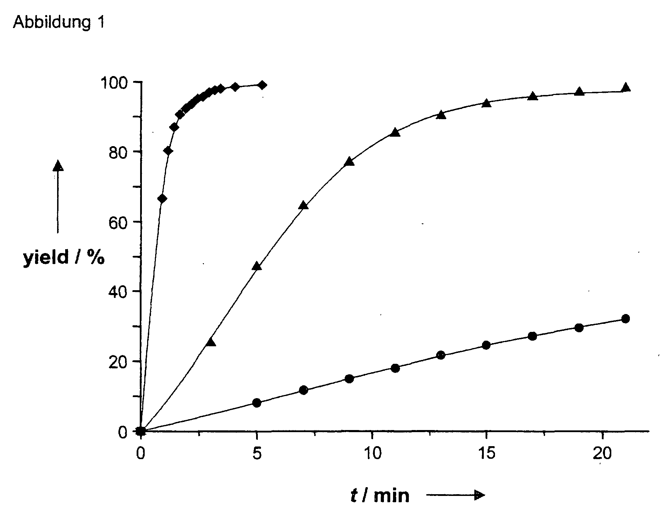

In einem NMR-Rohr werden 1.8 µmol der jeweiligen erfindungsgemäßen Komplexverbindung vorgelegt und in 0.55 ml CD₂Cl₂ gelöst (alternativ wird eine eingestellte Maßlösung verwendet). Anschließend wird die Polymerisationsreaktion durch Zugabe von 55 µl 1,5-Cyclooctadien (Monomer: Katalysator = 250 : 1) gestartet. Durch Aufnahme von ¹H-NMR-Spektren wird der Fortgang der Reaktion verfolgt.
Durch Integration der zeitabhängigen Signale von Produkt (Polycyclooctadienamer) und Edukt (Cyclooctadien) ergeben sich die zeitabhängigen Ausbeuten an Polycyclooctadienamer gemäß Abbildung 1 und die Turnover Frequencies (TOF), wie sie in Tab. 3 angegeben sind.

**Tab. 3**

| ROMP von 1,5-Cyclooctadien mit verschiedenen erfindungsgemäßen Komplexverbindungen unter NMR-Bedingungen. | | | |
|---|---|---|---|
| Komplex | Zeit [min] | Ausbeute [%] | TOF [h⁻¹] |
| 1 | 10.9 | 80 | 1100 |
| 2 | 9.1 | 80 | 1300 |
| 3 | 1.2 | 80 | 10000 |

Die hierbei erzielten TOFs liegen deutlich über denen bekannter Systeme. So weisen analoge Phosphansysteme, die gleichzeitig die aktivsten literaturbekannten Systeme auf Rutheniumbasis darstellen, unter gleichen Bedingungen TOFs von 200 bis maximal 1000 h⁻¹ auf.

### 2d) Ringöffnende Metathesepolymerisation von Cyclopenten

Ähnlich wie 1,5-Cyclooctadien gilt Cyclopenten als äußerst schwierig zu polymerisierendes Substrat.

### Typischer Versuchsansatz:

In einem Kolben werden 1.0 µmol der jeweiligen Komplexverbindung in 1 ml CH₂Cl₂ gelöst. Die Reaktion wird durch Zugabe von 5.0 mmol Cyclopenten gestartet und nach gewisser Zeit durch Eingießen der Reaktionslösung in 500 ml Methanol (Ausfällen des gebildeten Polycyclopentenamers) gestoppt. Das ausgefällte Polycyclopentenamer wird durch Filtration isoliert und nach mehrmaligem Umfällen aus CH₂Cl₂/ Methanol oder Toluol / Methanol im Hochvakuum bis zur Gewichtskonstanz getrocknet. Die Ausbeutebestimmung erfolgt gravimetrisch. Die Ergebnisse sind in Tab. 4 zusammengefasst.

**Tab. 4.**

| ROMP von Cyclopenten mit verschiedenen erfindungsgemäßen Komplexverbindungen. | | | | |
|---|---|---|---|---|
| Katalysator | Monomer / Katalysator | Zeit [min] | Ausbeute [%] | TOF [h⁻¹] |
| 1 | 5000 | 30 | 25 | 2500 |
| 2 | 5000 | 30 | 28 | 2800 |
| 3 | 5000 | 5 | 10 | 6000 |

### 3) Ringschlußmetathese

Das Potential der erfindungsgemäßen Komplexverbindungen in der Ringschlußmetathese wird durch die Reaktion von 1,7-Octadien zu Cyclohexen unter Freisetzung von Ethylen veranschaulicht (Tab. 5)

### Typischer Reaktionsansatz:

Eine Lösung von 6.3 µmol der jeweiligen Komplexverbindung in 2 ml 1,2-Dichlorethan wurde mit 0.45 mmol 1,7-Octadien versetzt. Nach 10 min bei 60 °C wurde das Reaktionsgemisch GC/MS-analytisch untersucht.

**Tab. 5**

| RCM von 1,7-Octadien mit verschiedenen erfindungsgemäßen Komplexverbindungen. | | | |
|---|---|---|---|
| Katalysator | Monomer / Katalysator | Zeit [min] | Ausbeute [%] |
| 1 | 50 | 10 | > 98 |
| 2 | 50 | 10 | > 98 |
| **3** | 50 | 10 | > 98 |

### 4) Metathese acyclischer Olefine

Das Potential der erfindungsgemäßen Komplexverbindungen in der Metathese acyclischer Olefine wird durch die Homometathese von 1-Octen zu 7-Tetradecen unter Freisetzung von Ethylen veranschaulicht (Tab. 6)

### Typischer Reaktionsansatz:

Eine Lösung von 6.0 µmol der jeweiligen Komplexverbindung in 1 ml 1,2-Dichlorethan wurde mit 3.0 mmol 1-Octen versetzt. Nach 3 h bei 45 °C wurde das Reaktionsgemisch GC/MS-analytisch untersucht.

**Tab. 6**

| Homometathese von 1-Octen mit verschiedenen erfindungsgemäßen Komplexverbindungen. | | | | |
|---|---|---|---|---|
| Katalysator | Monomer / Katalysator | Zeit [h] | Ausbeute [%] | E / Z |
| 1 | 500 | 3 | 51 | 3.7 |
| 2 | 500 | 3 | 47 | 3.4 |
| 3 | 500 | 3 | 22 | 3.5 |

ROMP von 1,5-Cyclooctadien mit erfindungsgemäßen Komplexverbindungen: ◇ Komplexverbindung 3; Δ Komplexverbindung 1; und o RuCl₂(PCy₃)₂(CHPh) (P. Schwab, M. B. France, J. W. Ziller, R. H. Grubbs, Angew. Chem., 1995, 107, 2179-2181; Angew. Chem. Int. Ed. Engl. 1995, 34, 2039-2041)

## Patentansprüche

1. Komplexverbindungen des Rutheniums der allgemeinen Strukturformel I 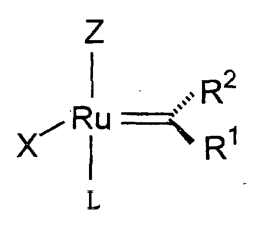 in der X ein anionischer Ligand ist,
in der Z einen ein- bis dreizähnigen Liganden darstellt, der ein Metall enthält und nicht-ionisch an das Rutheniumzentrum gebunden ist,
in der R¹ und R² gleich oder unabhängig voneinander verschieden sind, aber auch einen Cyclus ausbilden können,
in der R¹ und R² für Wasserstoff oder/und für eine Kohlenwasserstoffgruppe stehen,
wobei die Kohlenwasserstoffgruppen gleich oder unabhängig voneinander verschieden aus einem geradkettigen oder verzweigten oder/und cyclischen Rest aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen, Arylresten mit 6 bis 30 Kohlenstoffatomen und Silylresten bestehen,
wobei in den Kohlenwasserstoff- oder/und Silylgruppen die Wasserstoffatome teilweise oder gänzlich durch eine Alkyl-, Aryl-, Alkenyl-, Alkinyl-, Metallocenyl-, Halogen-, Nitro-, Nitroso-, Hydroxy-, Alkoxy-, Aryloxy-, Amino-, Amido-, Carboxyl-, Carbonyl-, Thio- oder/und Sulfonylgruppe einfach oder mehrfach, gleich oder unabhängig voneinander verschieden ersetzt sein können,
in der Ligand L ein N-heterocyclisches Carben der allgemeinen Formeln II - V ist, 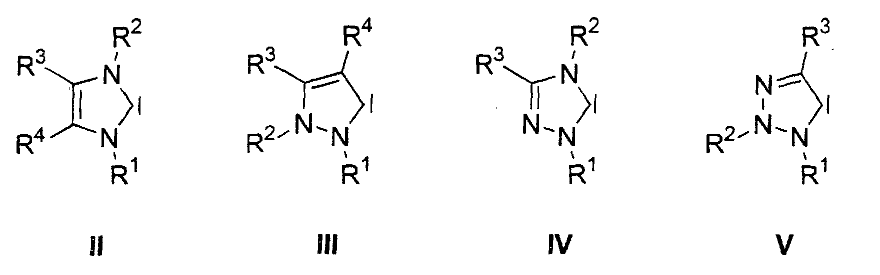 wobei R¹, R², R³ und R⁴ in den Formeln II, III, IV und V gleich oder unabhängig voneinander verschieden für Wasserstoff oder/und für eine Kohlenwasserstoffgruppe stehen,
wobei die Kohlenwasserstoffgruppen gleich oder unabhängig verschieden voneinander aus cyclischen, geradkettigen oder/und verzweigten Resten aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen, Arylresten mit 6 bis 30 Kohlenstoffatomen bestehen, bei denen gegebenenfalls mindestens ein Wasserstoff durch funktionelle Gruppen ersetzt sein kann, und wobei gegebenenfalls R³ und R⁴ für Halogen-, Nitro-, Nitroso-, Alkoxy-, Aryloxy-, Amido-, Carboxyl-, Carbonyl-, Thio- oder/und Sulfonylgruppe einfach oder mehrfach, gleich oder unabhängig voneinander verschieden stehen kann.

2. Komplexverbindungen nach Anspruch 1, **dadurch gekennzeichnet, daß** der anionische Ligand X ausgewählt ist unter Halogenid, Pseudohalogenid, Tetraphenylborat, perhalogeniertes Tetraphenylborat, Tetrahalogenoborat, Hexahalogenophosphat, Hexahalogenoantimonat, Trihalogenmethansulfonat, Alkoxid, Thiolat, Carboxylat, Tetrahalogenoaluminat, Tetracarbonylcobaltat, Hexahalogenoferrat(III), Tetrahalogenoferrat(III) und Tetrahalogenopalladat(II).

3. Komplexverbindungen nach Anspruch 1 oder 2, **dadurch gekennzeichnet, daß** Z einen ein- bis dreizähnigen Liganden L'ₙMX'ₘ darstellt,
wobei n = 0 bis 4, m = 0 bis 6 und m +n ≠ 0 ist,
wobei L' gleich oder unabhängig verschieden voneinander aus der Gruppe der π-gebundenen, ungesättigten carbocyclischen Kohlenwasserstoffe oder / und der Gruppe der neutralen Elektronendonoren ausgewählt ist,
wobei X' gleich oder unabhängig verschieden voneinander für einen anionischen Liganden aus der Gruppe von Halogenid, Pseudohalogenid, Tetraphenylborat, perhalogeniertes Tetraphenylborat, Tetrahalogenoborat, Hexahalogenophosphat, Hexahalogenoantimonat, Trihalogenmethansulfonat, Alkoxid, Thiolat, Carboxylat, Tetrahalogenoaluminat, Tetracarbonylcobaltat, Hexahalogenoferrat(III), Tetrahalogenoferrat(III) oder/und Tetrahalogenopalladat(II) steht,
und wobei M für ein Metall steht.

4. Komplexverbindungen nach Anspruch 3, **dadurch gekennzeichnet, daß** L' gleich oder unabhängig verschieden voneinander ausgewählt ist unter Cyclopentadienyl-, Pentamethylcyclopentadienyl- oder anders substituerten Cyclopentadienylrest, Benzol, substituerten Benzol, Phosphan, Phosphit, Amin, Imin, Nitril, N-heterocyclischen Carben und Carbonyl.

5. Komplexverbindungen nach Anspruch 3 oder 4, **dadurch gekennzeichnet, daß** X' gleich oder unabhängig verschieden voneinander ausgewählt ist unter einem Halogenid, Pseudohalogenid, Alkoxid, Thiolat und Carboxylat.

6. Komplexverbindungen nach einem der Ansprüche 3 bis 5, **dadurch gekennzeichnet, daß** M ein Metall der 1. bis 8. Nebengruppe oder der 1. bis 4. Hauptgruppe ist.

7. Komplexverbindungen nach Anspruch 6, **dadurch gekennzeichnet, daß** M ausgewählt ist unter Osmium, Rhodium, lridium, Ruthenium, Eisen, Palladium, Rhenium, Molybdän, Wolfram, Bor, Aluminium und Silicium.

8. Komplexverbindungen nach einem der Ansprüche 1 bis 7, **dadurch gekennzeichnet, daß** in den allgemeinen Formeln II, III, IV und V der Wasserstoff in den Kohlenwasserstoffgruppen R¹, R², R³ und R⁴ teilweise oder gänzlich durch Halogen-, Nitro-, Nitroso-, Hydroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden ersetzt sind.

9. Komplexverbindungen nach einem der Ansprüche 1 bis 8, **dadurch gekennzeichnet, daß** in den allgemeinen Formeln II, III, IV und V R³ und R⁴ ein anneliertes Ringsystem darstellen.

10. Verfahren zur Herstellung von acyclischen Olefinen mit zwei oder mehr Kohlenstoffatomen oder/und von cyclischen Olefinen mit drei oder mehr Kohlenstoffatomen jeweils entsprechend der allgemeinen Formel VI 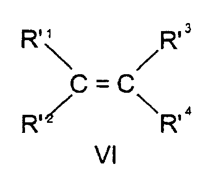 aus acyclischen Olefinen mit zwei oder mehr Kohlenstoffatomen oder/und aus cyclischen Olefinen mit drei oder mehr Kohlenstoffatomen jeweils entsprechend der allgemeinen Formel VI durch Olefin-Metathese-Reaktion in Gegenwart mindestens eines Katalysators, **dadurch gekennzeichnet, daß** ein Katalysator nach einem der Ansprüche 1 bis 9 eingesetzt wird und daß R'¹, R'², R'³ und R'⁴ in der allgemeinen Formel VI für Wasserstoff oder/und Kohlenwasserstoffgruppen stehen,
wobei die Kohlenwasserstoffgruppe gleich oder unabhängig voneinander verschieden aus geradkettigen, verzweigten oder/und cyclischen Resten aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen, Arylresten mit 6 bis 30 Kohlenstoffatomen, Metallocenyl- oder/und Silylresten besteht, bei denen gegebenenfalls mindestens ein Wasserstoff für eine funktionelle Gruppe stehen kann,
wobei gegebenenfalls R'¹, R'², R'³ und R'⁴ für Halogen-, Nitro-, Nitroso-, Hydroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden stehen.

11. Verfahren gemäß Anspruch 10 **dadurch gekennzeichnet, daß** in den eingesetzten oder/und herzustellenden Olefinen eine oder mehrere Doppelbindungen enthalten sind.

12. Verfahren gemäß Anspruch 10 oder 11, **dadurch gekennzeichnet,**
**daß** R'¹, R'², R'³ und R'⁴ in den Olefinen der allgemeinen Formel VI paarweise, gleich oder unabhängig voneinander verschieden, einen Cyclus ausbilden.

13. Verfahren gemäß einem der Ansprüche 10 bis 12, **dadurch gekennzeichnet, daß** in den Olefinen der allgemeinen Formel VI der Wasserstoff in den Kohlenwasserstoffgruppen R'¹, R'², R'³ und R'⁴ teilweise oder gänzlich durch Halogen-, Silyl-, Nitro-, Nitroso-, Hydroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden ersetzt sein können.

14. Verfahren nach einem der Ansprüche 10 bis 13, **dadurch gekennzeichnet, daß** der Prozeß in Lösungsmittel durchgeführt wird.

15. Verfahren nach Anspruch 14, **dadurch gekennzeichnet, daß** der Prozeß in organischen Lösungsmitteln durchgeführt wird.

16. Verfahren nach einem der Ansprüche 10 bis 15, **dadurch gekennzeichnet, daß** das Verfahren unter Zusatz einer Brönstedt-Säure durchgeführt wird.

17. Verfahren nach Anspruch 16, **dadurch gekennzeichnet,**
**daß** die Brönstedt-Säure ausgewählt ist unter HCl, HBr, Hl, HBF₄, HPF₆ und Trifluoressigsäure.

18. Verfahren nach einem der Ansprüche 10 bis 17, **dadurch gekennzeichnet, daß** das Verfahren unter Zusatz einer Lewis-Säure durchgeführt wird.

19. Verfahren nach Anspruch 18, **dadurch gekennzeichnet,**
**daß** die Lewis-Säure ausgewählt ist unter BF₃, AlCl₃, ZnCl₂ oder/und CuCl₂

20. Verwendung einer Komplexverbindung nach einem der Ansprüche 1 bis 9 in der Olefin-Metathese.

## Claims

1. Complex compounds of ruthenium of the general structural formula I 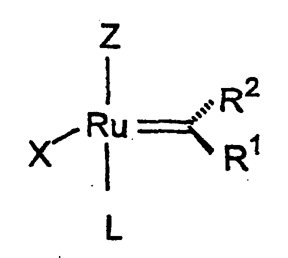 in which X is an anionic ligand,
in which Z is a mono- to tri-dentate ligand which comprises a metal and is bonded non-ionically to the ruthenium centre,
in which R¹ and R² are identical or different, independently of one another, but may also form a ring,
in which R¹ and R² represent hydrogen or/and a hydrocarbon group,
wherein the hydrocarbon groups are identical or different, independently of one another, and consist of a straight-chain or branched or/and cyclic radical from the group consisting of alkyl radicals having from 1 to 50 carbon atoms, alkenyl radicals having from 2 to 50 carbon atoms, alkynyl radicals having from 2 to 50 carbon atoms, aryl radicals having from 6 to 30 carbon atoms and silyl radicals,
wherein in the hydrocarbon or/and silyl groups some or all of the hydrogen atoms may be replaced by one or more identical or, independently of one another, different alkyl, aryl, alkenyl, alkynyl, metallocenyl, halogen, nitro, nitroso, hydroxy, alkoxy, aryloxy, amino, amido, carboxyl, carbonyl, thio or/and sulfonyl groups,
in which ligand L is an N-heterocyclic carbene of the general formula **II-V** 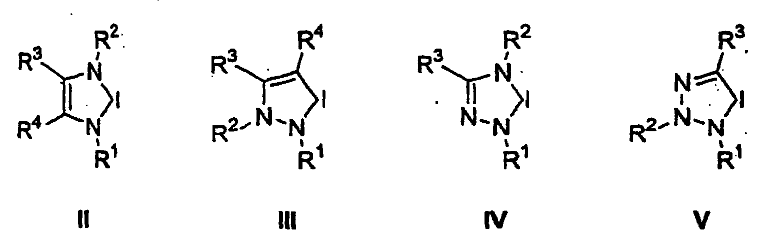 wherein R¹, R², R³ and R⁴ in formulae II, III, IV and V are identical or different, independently of one another, and represent hydrogen or/and a hydrocarbon group,
wherein the hydrocarbon groups are identical or different, independently of one another, and consist of cyclic, straight-chain or/and branched radicals from the group consisting of alkyl radicals having from 1 to 50 carbon atoms, alkenyl radicals having from 2 to 50 carbon atoms, alkynyl radicals having from 2 to 50 carbon atoms, aryl radicals having from 6 to 30 carbon atoms, in which at least one hydrogen may optionally be replaced by functional groups, and wherein R³ and R⁴ may optionally represent one or more identical or, independently of one another, different halogen, nitro, nitroso, alkoxy, aryloxy, amido, carboxyl, carbonyl, thio or/and sulfonyl groups.

2. Complex compounds according to claim 1, **characterised in that** the anionic ligand X is selected from halide, pseudohalide, tetraphenyl borate, perhalogenated tetraphenyl borate, tetrahaloborate, hexahalophosphate, hexahaloantimonate, trihalomethanesulfonate, alkoxide, thiolate, carboxylate, tetrahaloaluminate, tetracarbonyl cobaltate, hexahaloferrate(III), tetrahaloferrate(III) and tetrahalopalladate(II).

3. Complex compounds according to claim 1 or 2,
**characterised in that** Z represents a mono- to tri-dentate ligand L'ₙMX'ₘ,
wherein n = from 0 to 4, m = from 0 to 6 and m + n ≠ 0,
wherein L' may be identical or different, independently of one another, and is elected from the group of the π-bonded,
unsaturated carbocyclic hydrocarbons or/and the group of the neutral electron donors,
wherein X' may be identical or different, independently of one another, and represents an anionic ligand from the group consisting of halide, pseudohalide, tetraphenyl borate, perhalogenated tetraphenyl borate, tetrahaloborate, hexahalophosphate, hexahaloantimonate, trihalomethanesulfonate, alkoxide, thiolate, carboxylate, tetrahaloaluminate, tetracarbonyl cobaltate, hexahaloferrate(III), tetrahaloferrate(III) or/and tetrahalopalladate(II),
and wherein M represents a metal.

4. Complex compounds according to claim 3, **characterised in that** L' may be identical or different, independently of one another, and is selected from cyclopentadienyl, pentamethylcyclopentadienyl or a differently substituted cyclopentadienyl radical, benzene, substituted benzene, phosphane, phosphite, amine, imine, nitrile, N-heterocyclic carbene and carbonyl.

5. Complex compounds according to claim 3 or 4, **characterised in that** X' may be identical or different, independently of one another, and is selected from a halide, pseudohalide, alkoxide, thiolate and carboxylate.

6. Complex compounds according to any one of claims 3 to 5, **characterised in that** M is a metal of sub-group 1 to 8 or of main group 1 to 4.

7. Complex compounds according to claim 6, **characterised in that** M is selected from osmium, rhodium, iridium, ruthenium, iron, palladium, rhenium, molybdenum, tungsten, boron, aluminium and silicon.

8. Complex compounds according to any one of claims 1 to 7, **characterised in that** in the general formulae II, III, IV and V some or all of the hydrogens in the hydrocarbon groups R¹, R², R³ and R⁴ have been replaced by one or more identical or, independently of one another, different halogen, nitro, nitroso, hydroxy, alkoxy, aryloxy, amino, amido, carboxyl, carbonyl, thio, sulfonyl or/and metallocenyl groups.

9. Complex compounds according to any one of claims 1 to 8, **characterised in that** in the general formulae II, III, IV and V R³ and R⁴ represent a fused ring system.

10. Process for the preparation of acyclic olefins having two or more carbon atoms or/and of cyclic olefins having three or more carbon atoms, in each case corresponding to the general formula VI 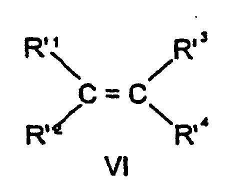 from acyclic olefins having two or more carbon atoms or/and from cyclic olefins having three or more carbon atoms, in each case corresponding to the general formula VI, by olefin metathesis reaction in the presence of at least one catalyst, **characterised in that** a catalyst according to any one of claims 1 to 9 is used and **in that** R'¹ R'², R'³ and R'⁴ in the general formula VI represent hydrogen or/and hydrocarbon groups,
wherein the hydrocarbon groups are identical or different, independently of one another, and consist of straight-chain, branched or/and cyclic radicals from the group consisting of alkyl radicals having from 1 to 50 carbon atoms, alkenyl radicals having from 2 to 50 carbon atoms, alkynyl radicals having from 2 to 50 carbon atoms, aryl radicals having from 6 to 30 carbon atoms, metallocenyl radicals or/and silyl radicals, in which at least one hydrogen may optionally represent a functional group,
wherein R'¹, R'², R'³ and R'⁴ may optionally represent one or more identical or, independently of one another, different halogen, nitro, nitroso, hydroxy, alkoxy, aryloxy, amino, amido, carboxyl, carbonyl, thio, sulfonyl or/and metallocenyl groups.

11. Process according to claim 10, **characterised in that** one or more double bonds are present in the olefins that are used or/and are to be prepared.

12. Process according to claim 10 or 11, **characterised in that** pairs of R'¹, R'², R'³ and R'⁴, identical or different, independently of one another, in the olefins of the general formula VI form a ring.

13. Process according to any one of claims 10 to 12, **characterised in that** in the olefins of the general formula VI some or all of the hydrogens in the hydrocarbon groups R'¹, R'², R'³ and R'⁴ may be replaced by one or more identical or, independently of one another, different halogen, silyl, nitro, nitroso, hydroxy, alkoxy, aryloxy, amino, amido, carboxyl, carbonyl, thio, sulfonyl or/and metallocenyl groups.

14. Process according to any one of claims 10 to 13, **characterised in that** the process is carried out in a solvent.

15. Process according to claim 14, **characterised in that** the process is carried out in organic solvents.

16. Process according to any one of claims 10 to 15, **characterised in that** the process is carried out with the addition of a Brönstedt acid.

17. Process according to claim 16, **characterised in that** the Brönstedt acid is selected from HCl, HBr, HI, HBF₄, HPF₆ and trifluoroacetic acid.

18. Process according to any one of claims 10 to 17, **characterised in that** the process is carried out with the addition of a Lewis acid.

19. Process according to claim 18, **characterised in that** the Lewis acid is selected from BF₃, AlCl₃, ZnCl₂ or/and CuCl₂.

20. Use of a complex compound according to any one of claims 1 to 9 in olefin metathesis.

## Revendications

1. Composés complexes du ruthénium de formule de structure générale I 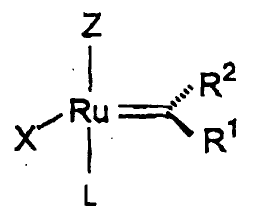 dans laquelle X est un ligand anionique,
dans laquelle Z représente un ligand monodentate à tridentate, qui contient un métal et qui est lié de manière non ionique au centre ruthénium,
dans laquelle R¹ et R² sont identiques ou différents, indépendamment l'un de l'autre, mais peuvent également former un cycle,
dans laquelle R¹ et R² représentent hydrogène et/ou un groupe hydrocarboné, les groupes hydrocarbonés, de manière identique ou différente, indépendamment l'une de l'autre, étant constitués par un radical linéaire ou ramifié et/ou cyclique du groupe des radicaux alkyle comprenant 1 à 50 atomes de carbone, des radicaux alcényle comprenant 2 à 50 atomes de carbone, des radicaux alcynyle comprenant 2 à 50 atomes de carbone, des radicaux aryle comprenant 6 à 30 atomes de carbone et des radicaux silyle,
les atomes d'hydrogène dans les groupes hydrocarbonés et/ou silyle pouvant être remplacés partiellement ou totalement, une fois ou plusieurs fois, de manière identique ou différente, indépendamment l'une de l'autre, par un groupe alkyle, aryle, alcényle, alcynyle, métallocényle, halogène, nitro, nitroso, hydroxy, alcoxy, aryloxy, amino, amido, carboxyle, carbonyle, thio et/ou sulfonyle,
dans laquelle le ligand L est un carbone N-hétérocyclique des formules générales II à V : 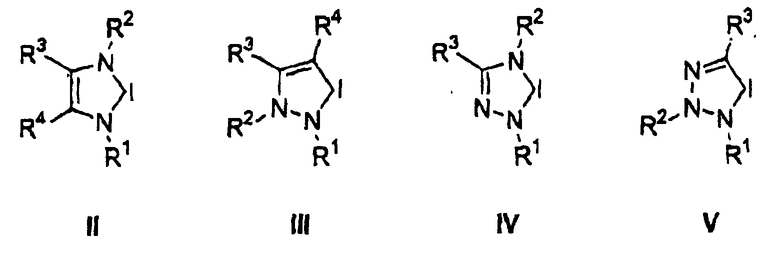 où R¹, R², R³ et R⁴ dans les formules II, III, IV et V, de manière identique ou différente, indépendamment l'une de l'autre, représentent hydrogène et/ou un groupe hydrocarboné, les groupes hydrocarbonés, de manière identique ou différente, indépendamment l'une de l'autre, étant constitués par des radicaux cycliques, linéaires et/ou ramifiés du groupe des radicaux alkyle comprenant 1 à 50 atomes de carbone, des radicaux alcényle comprenant 2 à 50 atomes de carbone, des radicaux alcynyle comprenant 2 à 50 atomes de carbone, des radicaux aryle comprenant 6 à 30 atomes de carbone, dans lesquels le cas échéant au moins un hydrogène peut être remplacé par des groupes fonctionnels et où, le cas échéant R³ et R⁴, une fois ou plusieurs fois, de manière identique ou différente, indépendamment l'une de l'autre, représentent un groupe halogène, nitro, nitroso, alcoxy, aryloxy, amido, carboxyle, carbonyle, thio et/ou sulfonyle.

2. Composés complexes selon la revendication 1, **caractérisés en ce que** le ligand anionique X est choisi parmi halogénure, pseudohalogénure, tétraphénylborate, tétraphénylborate perhalogéné, tétrahalogénoborate, hexahalogénophosphate, hexahalogénoantimonate, trihalogénométhanesulfonate, alcoxyde, thiolate, carboxylate, tétrahalogénoaluminate, tétracarbonylcobaltate, hexahalogénoferrate (III), tétrahalogénoferrate (III) et tétrahalogénopalladate (II).

3. Composés complexes selon la revendication 1 ou 2, **caractérisés en ce que** Z représente un ligand monodentate à tridentate L'ₙMX'ₘ
où n = 0 à 4, m = 0 à 6 et m + n ≠ 0,
où L', de manière identique ou différente, indépendamment l'une de l'autre, est sélectionné du groupe des hydrocarbures liés par une liaison n, insaturés carbocycliques et/ou du groupe des donneurs d'électrons neutres,
où X', de manière identique ou différente, indépendamment l'une de l'autre, représente un ligand anionique du groupe halogénure, pseudohalogénure, tétraphénylborate, tétraphénylborate perhalogéné, tétrahalogénoborate, hexahalogénophosphate, hexahalogénoantimonate, trihalogénométhanesulfonate, alcoxyde, thiolate, carboxylate, tétrahalogénoaluminate, tétracarbonylcobaltate, hexahalogénoferrate (III), tétrahalogénoferrate (III) et/ou tétrahalogénopalladate (II) et
où M représente un métal.

4. Composés complexes selon la revendication 3, **caractérisés en ce que** L', de manière identique ou différente, indépendamment l'une de l'autre, est choisi parmi les radicaux cyclopentadiényle, pentaméthylcyclopentadiényle ou un radical cyclopentadiényle substitué différemment, benzène, benzène substitué, phosphane, phosphite, amine, imine, nitrile, carbone et carbonyle N-hétérocyclique.

5. Composés complexes selon la revendication 3 ou 4, **caractérisés en ce que** X, de manière identique ou différente, indépendamment l'une de l'autre, est choisi parmi un halogénure, un pseudohalogénure, un alcoxyde, un thiolate et un carboxylate.

6. Composés complexes selon l'une quelconque des revendications 3 à 5, **caractérisés en ce que** M est un métal du 1er au 8ème groupe secondaire ou du 1er au 4ème groupe principal.

7. Composés complexes selon la revendication 6, **caractérisés en ce que** M est choisi parmi l'osmium, le rhodium, l'iridium, le ruthénium, le fer, le palladium, le rhénium, le molybdène, le tungstène, le bore, l'aluminium et le silicium.

8. Composés complexes selon l'une quelconque des revendications 1 à 7, **caractérisés en ce que** dans les formules générales II, III, IV et V, les atomes d'hydrogène dans les groupes hydrocarbonés R¹, R², R³ et R⁴ sont remplacés partiellement ou totalement, une fois ou plusieurs fois, de manière identique ou différente, indépendamment l'une de l'autre, par des groupes halogène, nitro, nitroso, hydroxy, alcoxy, aryloxy, amino, amido, carboxyle, carbonyle, thio, sulfonyle et/ou métallocényle.

9. Composés complexes selon l'une quelconque des revendications 1 à 8, **caractérisés en ce que** dans les formules générales II, III, IV et V, R³ et R⁴ représentent un système cyclique annelé.

10. Procédé pour la préparation d'oléfines acycliques comprenant deux atomes de carbone ou plus et/ou d'oléfines cycliques comprenant trois atomes de carbone ou plus, correspondant à chaque fois à la formule générale VI 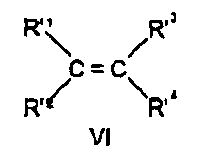 à partir d'oléfines acycliques comprenant deux atomes de carbone ou plus et/ou à partir d'oléfines cycliques comprenant trois atomes de carbone ou plus, correspondant à chaque fois à la formule générale VI, par une réaction de métathèse d'oléfines en présence d'au moins un catalyseur, **caractérisé en ce qu'**on utilise un catalyseur selon l'une quelconque des revendications 1 à 9 et **en ce que** R^{'1}, R^{'2}, R^{'3} et R^{'4} dans la formule générale VI représentent hydrogène et/ou des groupes hydrocarbonés, les groupes hydrocarbonés, de manière identique ou différente, indépendamment l'une de l'autre pouvant être constitués par des radicaux linéaires, ramifiés et/ou cycliques du groupe des radicaux alkyle comprenant 1 à 50 atomes de carbone, des radicaux alcényle comprenant 2 à 50 atomes de carbone, des radicaux alcynyle comprenant 2 à 50 atomes de carbone, des radicaux aryle comprenant 6 à 30 atomes de carbone, des radicaux métallocényle et/ou des radicaux silyle, dans lesquels le cas échéant au moins un hydrogène peut représenter un groupe fonctionnel,
où, le cas échéant, R^{'1}, R^{'2}, R^{'3} et R^{'4}, une fois ou plusieurs fois, de manière identique ou différente, indépendamment l'une de l'autre, représentent des groupes halogène, nitro, nitroso, hydroxy, alcoxy, aryloxy, amino, amido, carboxyle, carbonyle, thio, sulfonyle et/ou métallocényle.

11. Procédé selon la revendication 10, **caractérisé en ce que** dans les oléfines utilisées et/ou à préparer, une ou plusieurs doubles liaisons sont contenues.

12. Procédé selon la revendication 10 ou 11, **caractérisé en ce que** R^{1'}, R^{'2}, R^{'3} et R^{'4} dans les oléfines de formule générale VI, de manière identique ou différente, indépendamment l'une de l'autre, forment par paire un cycle.

13. Procédé selon l'une quelconque des revendications 10 à 12, **caractérisé en ce que** dans les oléfines de formule générale VI, l'hydrogène dans les groupes hydrocarbonés R^{'1}, R^{'2}, R'³ et R^{'4} peut être remplacé partiellement ou totalement, une fois ou plusieurs fois, de manière identique ou différente, indépendamment l'un de l'autre, par des groupes halogène, silyle, nitro, nitroso, hydroxy, alcoxy, aryloxy, amino, amido, carboxyle, carbonyle, thio, sulfonyle et/ou métallocényle.

14. Procédé selon l'une quelconque des revendications 10 à 13, **caractérisé en ce que** le processus est réalisé dans un solvant.

15. Procédé selon la revendication 14, **caractérisé en ce que** le processus est réalisé dans des solvants organiques.

16. Procédé selon l'une quelconque des revendications 10 à 15, **caractérisé en ce que** le procédé est réalisé avec addition d'un acide de Brönstedt.

17. Procédé selon la revendication 16, **caractérisé en ce que** l'acide de Brönstedt est choisi parmi HCl, HBr, HI, HBF₄, HPF₆ et l'acide trifluoroacétique.

18. Procédé selon l'une quelconque des revendications 10 à 17, **caractérisé en ce que** le procédé est réalisé avec addition d'un acide de Lewis.

19. Procédé selon la revendication 18, **caractérisé en ce que** l'acide de Lewis est choisi parmi BF₃, AlCl₃, ZnCl₂ et/ou CuCl₂.

20. Utilisation d'un composé complexe selon l'une quelconque des revendications 1 à 9 dans la métathèse d'oléfines.
